# EUROPEAN PATENT APPLICATION

(11) **EP 2 623 191 A1**
(43) Date of publication of application: **07.08.2013**
(21) Application number: 11829110.3
(22) Date of filing: 27.09.2011
(51) Int. Cl.: B01F 5/00, B01F 15/06, B01J 19/00, B81B 1/00, F28F 3/00

(54) **MICROMIXER**

(30) Priority: 30.09.2010 JP 2010221208
(71) Applicant: DIC Corporation, Tokyo 174-8520 (JP)
(72) Inventor: ISHIYAMA Fumihiko, Sakura-shi, Chiba 258-8668 (JP); HIZAWA Takeshi, Sakura-shi, Chiba 285-8668 (JP); NAKAMURA Masayuki, Sakura-shi, Chiba 285-8668 (JP)
(74) Representative: Albrecht, Thomas
(86) International application number: PCT/JP2011/072069
(87) International publication number: WO 2012/043557

(57) **Abstract**

A micromixer includes a stack unit in which a first plate and a second plate are stacked, the first plate having a first microtubular flow path connected to a fluid supply path through which a first fluid flows, the second plate having a second microtubular flow path through which a second fluid flows and which is connected to a fluid supply path through which the second fluid flows; and a mixing space which is connected to an outlet of the first microtubular flow path and an outlet of the second microtubular flow path and in which the first fluid and the second fluid are mixed. In at least one of the first plate and the second plate, the microtubular flow path has an inlet portion connected to the fluid supply path and constituted by a single flow path, and an outlet portion connected to the mixing space, and a cross-sectional area of the fluid that liquid-tightly flows through the microtubular flow path in the outlet portion is smaller than a cross-sectional area of the fluid that liquid-tightly flows through the single microtubular flow path in the inlet portion.

## Description

### Technical Field

The present invention relates to a micromixer that can mix fluids in a stable feeding state even in the case where high-viscosity fluids are mixed with each other or even in the case where a low-viscosity fluid is mixed with a fluid having a viscosity that is about 3,000 times higher than the viscosity of the low-viscosity fluid, without the low-viscosity fluid being intermittently fed.

### Background Art

Various types of static mixers have been proposed for the purpose of mixing at least two types of fluids in a step of producing fine particles using crystallization or the like or in a step of a chemical reaction. Among such static mixers, micromixers in which fluids to be mixed are supplied to micro-flow paths have attracted attention as efficient mixing devices.

A micromixer has a mechanism in which at least two types of fluids are divided into fine flows in micro-flow paths each having a flow path width of about 10 to 1,000 µm and are then mixed with each other. When the fluids supplied into the micromixer are divided into fine flows, the diffusion lengths of the fluids are reduced, and the mixing rate of the fluids increases. Thus, the fluids supplied into the micromixer are efficiently mixed within a short time compared with existing static mixers.

As an example of the structure of a micromixer, a mixer having a Y-letter shaped flow path (Y-shaped micromixer) is known. The Y-shaped micromixer has a structure in which a flow path through which a first fluid flows and a flow path through which a second fluid flows intersect on a plate at an acute angle and then join together to form a single flow path, that is, to form a Y-letter shape. The fluids supplied to this mixer join at the intersecting portion of the flow paths in a laminar flow state, and are mixed by interdiffusion.

In the case where a low-viscosity fluid and a fluid (different-viscosity fluid) having a viscosity that is about 10 times higher than that of the low-viscosity fluid are allowed to flow through the Y-shaped micromixer at the same flow rate, the two types of fluids flow such that the pressure losses thereof become the same. More specifically, the flow rate of the low-viscosity fluid becomes high, and the proportion of the cross section of a micro-flow path that is used decreases. The reverse phenomenon occurs in the high-viscosity fluid. Consequently, the pressure losses of the two fluids become the same. In the case where the difference in viscosity is large to some extent, the low-viscosity fluid flows through an extremely small cross section at a high flow rate, which may cause an unstable flow such as intermittent feeding. Therefore, fluids cannot stably flow, resulting in a problem that the fluids cannot be uniformly mixed.

Even in the case where the cross-sectional area of the micro-flow path in the confluence portion is increased in accordance with the difference in viscosity between two fluids, the interdiffusion time of the liquids increases, thereby decreasing a mixing property of the fluids. In this manner, in the interdiffusion of liquids in a micro-flow path, problems negligibly occur in the case where low-viscosity fluids are mixed with each other. However, in the case of fluids having different viscosities or in the case of high-viscosity fluids, the problem of a mixing property being decreased may often occur.

An example of a micromixer other than the Y-shaped micromixer is a stacked-type micromixer having a structure in which a plate having fine channels through which a reactant A to be mixed flows and a plate having fine channels through which a reactant B flows are stacked (refer to, for example, PTL 1). The fine channels of the stacked-type micromixer are each arranged so as to form an acute angle when viewed from the top surface of the plate. The fluids are combined in a confluence chamber provided at outlets of the fine channels.

The micromixer (outlet mixing-type micromixer) described in PTL 1, the micromixer having the stacked structure and including the confluence chamber for combining fluids, has a structure in which micro-flow paths are formed in each of the two plates stacked in the vertical direction and the micro-flow paths intersect toward a mixing chamber, which is a common outlet. In addition, the width and the depth of the flow paths are each 250 µm or less. Thus, this micromixer targets fluids that have low viscosities and that are easily, instantaneously mixed, and is not a mixer used for mixing high-viscosity fluids with each other or mixing fluids having different viscosities with each other. Therefore, in the case where high-viscosity fluids or fluids having different viscosities flow through the mixer described in PTL 1, a pressure loss, clogging, etc. occur and it becomes difficult to allow the fluids to flow. Consequently, mixing may not be sufficiently performed.

### Citation List

### Patent Literature

PTL 1: Japanese Unexamined Patent Application Publication (Translation of PCT Application) No. 9-512742 (page 12 and Fig. 3)

### Summary of Invention

### Technical Problem

A first object of the present invention is to provide a micromixer that can uniformly mix fluids with a good mixing efficiency even in the case where high-viscosity fluids are mixed with each other or even in case where a low-viscosity fluid is mixed with a fluid having a viscosity that is about 3,000 times higher than that of the low-viscosity fluid. A second object of the present invention is to provide a method for obtaining a liquid mixture of fluids having significantly different viscosities using the micromixer. A third object of the present invention is to provide a method for producing a compound by using the method for obtaining a liquid mixture.

### Solution to Problem

The inventors of the present invention conducted intensive studies in order to achieve the above objects. As a result, the inventors of the present invention found the following: In a stacked-type micromixer having a structure in which a plate having a fine channel through which a reactant A to be mixed flows and a plate having a fine channel through which a reactant B flows are stacked, in at least one of the plates, the microtubular flow path has an inlet portion connected to the fluid supply path and constituted by a single flow path, and a cross-sectional area of the microtubular flow path in an outlet portion connected to a mixing space is smaller than a cross-sectional area of the microtubular flow path in the inlet portion. With this structure, even in the case where high-viscosity fluids are mixed with each other or even in the case where a low-viscosity fluid is mixed with a fluid having a viscosity that is about 3,000 times higher than that of the low-viscosity fluid, the mixing can be performed with a good mixing efficiency while the low-viscosity fluid is stably fed without being intermittently fed. Furthermore, compounds obtained by using raw materials having significantly different viscosities, namely, a urethane meth(acrylate) and pigment fine particles having an anthraquinone skeleton, can be suitably produced by using this micromixer. This finding resulted in the completion of the present invention.

Specifically, the present invention provides a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows; and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion.

The present invention provides a method for producing a liquid mixture using a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion, the method including allowing the first fluid and the second fluid that have a viscosity ratio of 10 to 3,000 to respectively flow through the first microtubular flow path and the second microtubular flow path to obtain a fluid mixture containing the first fluid and the second fluid in the mixing space.

The present invention provides a method for producing a urethane (meth)acrylate using a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion, the method including:
a first step of allowing a compound (A) having an isocyanate group to flow through the first microtubular flow path of the first plate and allowing a (meth)acrylate (B) having a hydroxyl group to flow through the second microtubular flow path of the second plate to obtain a fluid mixture containing the compound (A) and the compound (B) in the mixing space; and
a second step of allowing the compound (A) and the compound (B) in the fluid mixture to react with each other.

The present invention further provides a method for producing pigment fine particles having an anthraquinone structure using a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion, the method including:
a first step of mixing water (C) and a sulfuric acid solution (D) of a pigment having an anthraquinone structure in the mixing space by allowing the water (C) to flow through the first microtubular flow path of the first plate and allowing the sulfuric acid solution (D) to flow through the second microtubular flow path of the second plate to obtain a fluid mixture in which the pigment having an anthraquinone structure is deposited; and
a second step of cooling the fluid mixture in which the pigment is deposited.

### Advantageous Effects of Invention

According to the micromixer of the present invention, a micro-flow path is formed such that a cross-sectional area of a fluid in an outlet portion of the microtubular flow path is smaller than a cross-sectional area of the fluid in an inlet portion of the microtubular flow path. Therefore, the fluid flowing through the micro-flow path is not affected by the pressure loss, and the flow rate of the fluid can be increased. The fluid forms a turbulent flow, and the mixing efficiency can be improved. According to the micromixer of the present invention, in the case where, for example, high-viscosity fluids are mixed with each other or a low-viscosity fluid is mixed with a fluid having a viscosity significantly higher than the viscosity of the low-viscosity fluid, the fluids can be mixed in a state of stable feeding without causing intermittent feeding of the low-viscosity fluid. By using the micromixer of the present invention, compounds obtained by using raw materials having significantly different viscosities, for example, a urethane meth(acrylate) and pigment fine particles having an anthraquinone structure can be easily produced. In particular, pigment fine particles having an anthraquinone structure and having a small average particle diameter of about 10 nm, the pigment fine particles having hitherto been difficult to be produced, can be obtained by the production method of the present invention. The pigment fine particles having a small particle diameter are particularly useful for a red color filter having a high contrast.

### Brief Description of Drawings

[Fig. 1] Fig. 1 is a schematic view of a micromixer 1 according to an embodiment.
[Fig. 2] Fig. 2 is a schematic view of a micromixer 2 according to another embodiment.
[Fig. 3] Fig. 3 is a schematic view of a micromixer 3 according to another embodiment.
[Fig. 4] Fig. 4 is an exploded perspective view of a stacked body included in the micromixer 1.
[Fig. 5] Fig. 5 is a schematic view of a first plate which is a component of the micromixer 1.
[Fig. 6] Fig. 6 includes schematic views of other examples of a first plate which is a component of the micromixer 1.
[Fig. 7] Fig. 7 is a schematic view of another example of a first plate which is a component of the micromixer 1.
[Fig. 8] Fig. 8 includes schematic views of other examples of a first plate which is a component of the micromixer 1.
[Fig. 9] Fig. 9 is a perspective view of a second plate which is a component of the micromixer 1.
[Fig. 10] Fig. 10 includes schematic views of other examples of a second plate which is a component of the micromixer 1.
[Fig. 11] Fig. 11 is an exploded perspective view of a stacked body including plates each having a flow path through which a heat exchange medium flows.
[Fig. 12] Fig. 12 is a schematic view of another example of a micromixer.
[Fig. 13] Fig. 13 is a schematic block diagram illustrating a device used in Examples.
[Fig. 14] Fig. 14 is a schematic view of a microreactor for a reaction used in Examples.
[Fig. 15] Fig. 15 is an exploded perspective view of a stacked body included in a microreactor for a reaction used in Examples.
[Fig. 16] Fig. 16 is an exploded perspective view of another example of a stacked body including plates each having a flow path through which a heat exchange medium flows.
[Fig. 17] Fig. 17 is a schematic block diagram illustrating a device used in Examples.

### Description of Embodiments

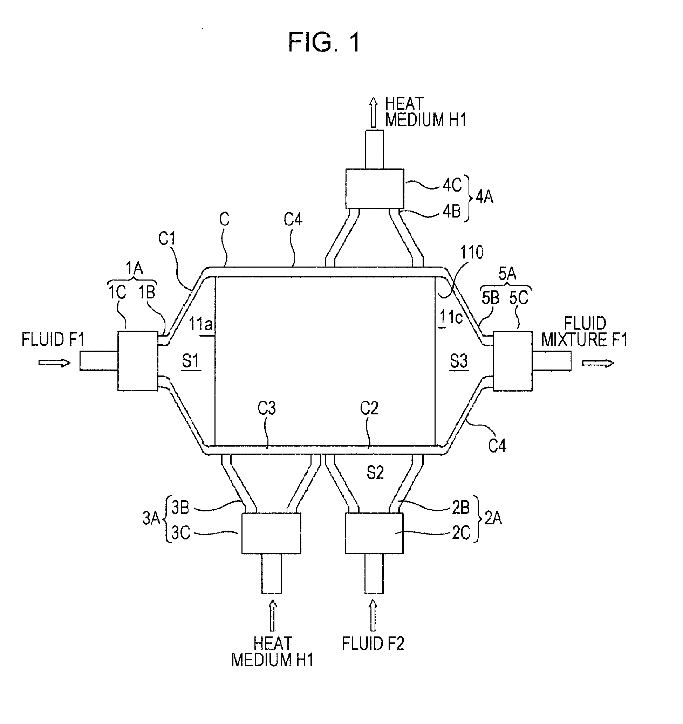

Embodiments of the present invention will now be described with reference to Figs. 1 to 17. Fig. 1 is a schematic view illustrating an example of a micromixer 1.

The micromixer 1 includes a hollow case C. In this case C, a stacked body 110 including a stack unit is fixed in place. The stack unit includes a first plate having a first microtubular flow path connected to a fluid supply path through which a first fluid (F1) flows, and a second plate having a second microtubular flow path through which a second fluid (F2) flows and which is connected to a fluid supply path through which the second fluid flows, the second plate being stacked on the first plate.

In the micromixer of the present invention, the stack unit, in which the first plate and the second plate are stacked, may further include a temperature control plate having a heat exchange medium flow path through which a heat exchange medium flows, as exemplified in the micromixer 1. The temperature control plate is preferably stacked so that the first-flow-path-forming portion and the first medium flow path are located so as to correspond to each other in the stacking direction and the second-flow-path-forming portion and the second medium flow path are located so as to correspond to each other in the stacking direction. This structure is preferable from the standpoint of achieving good heat exchange efficiency.

Furthermore, the stacked body 110 preferably includes a temperature control plate having a heat exchange medium flow path through which a heat medium H11, which performs heat exchange between the fluid F1 and the fluid F2, flows because the temperature of the fluid F1 and the temperature of the fluid F2 can be made uniform and a decrease in the mixing efficiency due to the difference in temperature between the fluid F1 and the fluid F2 can be suppressed.

A first fluid supply unit 1A that supplies the first fluid (F1) to the case C is provided at a left end C1 of the case C of the micromixer 1. A second fluid supply unit 2A that supplies the second fluid (F2) to the case C is provided at a lower right end C2 of the case C. Hereinafter, in the case where the fluid supply unit 1A and the fluid supply unit 2B are described without distinction, they are each simply described as a fluid supply unit 1.

The fluid supply unit 1A includes an opening 1B formed at a left end portion of the case C and a connector 1C connected to the opening 1B. The connector 1C is connected to a fluid supply path through which the first fluid (F1) flows. Accordingly, this fluid supply path is connected to the first microtubular flow path of the first plate. The fluid supply path is connected to a pressure-feeding mechanism including a tank that stores the first fluid (F1), a booster pump, a pipe line connected to the pump, etc. The first fluid (F1) is fed to the connector 1C side by the mechanism in a pressurized state. A space is provided between the opening 1B and a side face 11a of the stacked body 110 fixed in the case C. The space functions as a storage portion S1 that temporarily stores the first fluid (F1) fed from the pressure-feeding mechanism.

The fluid supply unit 2A includes an opening 2B formed at the lower right end of the case C and a connector 2C connected to the opening 2B. The connector 2C is connected to a fluid supply path through which the second fluid (F2) flows. Accordingly, this fluid supply path is connected to the second microtubular flow path of the second plate. The fluid supply path is connected to a pressure-feeding mechanism including a tank that stores the second fluid (F2), a booster pump, a pipe line connected to the pump, etc. The second fluid (F2) is fed to the connector 3B side by the mechanism in a pressurized state. A space is provided between the opening 1B and a side face 11b of the stacked body 110 fixed in the case C. The space functions as a storage portion S2 that temporarily stores the second fluid (F2) fed from the pressure-feeding mechanism.

A heat medium supply unit 3A that supplies a heat medium H1 into the case C is formed at a lower left end C3 of the case C. Similarly to the fluid supply units 1A and 2A, the heat medium supply unit 3A includes an opening 3B and a connector 3C. The heat medium H1 supplied to the heat medium supply unit 3A passes through a flow path formed in the stacked body 110, and is sent out from a heat medium-sending unit 4A formed at an upper end C4 of the case C to the outside of the case C. Similarly to the fluid supply units 1A and 2A, the heat medium-sending unit 4A includes an opening 4B and a connector 4C.

A sending unit 5A including an opening 5B formed at a right end portion of the case C and a connector 5C connected to the opening 5B is provided on a right end C4 of the case C. A space is provided between the opening 5B and a side face 11c of the stacked body 110 fixed in the case C. The space is connected to an outlet of the first microtubular flow path and an outlet of the second microtubular flow path and functions as a mixing space S3 in which the first fluid and the second fluid are mixed. The volume of the S3 is determined in consideration of the pressure loss to be generated, stable flows of high-viscosity fluids and fluids having different viscosities, the mixing power, and the strength of the device. The volume of the mixing space S3 can be changed in accordance with the viscosities of the fluids, the desired degree of mixing, etc. The volume of the S3 is preferably 100 mm³ or more, and more preferably 250 mm³ or more from the standpoint that a stable flow can be realized particularly when two or more fluids having different viscosities (different-viscosity fluids) are allowed to flow and mixed.

In order to realize a stable flow in the case where the difference in viscosity between the first fluid and the second fluid is large, for example, 10 times or more, the cross-sectional area of the mixing space S3 is preferably made large because a uniform mixture of the first fluid and the second fluid can be obtained. In addition, in the case where the difference in viscosity between the first fluid and the second fluid is large and the flow rate of a high-viscosity fluid is higher than the flow rate of a low-viscosity fluid, it is preferable to further increase the cross-sectional area of the mixing space.

The area of a cross section at which the mixing space S3 is connected to the outlet of the first microtubular flow path and the outlet of the second microtubular flow path is preferably 50 mm² or more and more preferably 100 mm² or more because it is possible to obtain a mixture in which the first fluid and the second fluid are sufficiently mixed.

In the micromixer, the area of the cross section at which the mixing space S3 is connected to the outlet of the first microtubular flow path and the outlet of the second microtubular flow path is preferably 5 to 50 times the sum of the cross-sectional area of the outlet of the first microtubular flow path and the cross-sectional area of the outlet of the second microtubular flow path from the standpoint that a low-viscosity fluid out of fluids having different viscosities does not flow intermittently but can flow stably and satisfactory mixing can be achieved. The area of the cross section is more preferably 10 to 200 times, and still more preferably 20 to 300 times the sum of the cross-sectional area of the outlet of the first microtubular flow path and the cross-sectional area of the outlet of the second microtubular flow path.

As described above, the micromixer of the present invention can be suitably used in the mixing of high-viscosity fluids or the mixing of fluids having significantly different viscosities. In the micromixer of the present invention, the first fluid and the second fluid that have respectively flown through the microtubular flow paths of the first plate and the second plate flow into the mixing space. The resulting fluid that has flown into the mixing space is discharged from the sending unit 5A in Fig. 1 to the outside of the micromixer. The outlet velocity of the fluid at this time becomes lower than the velocity when the first and second fluids flow into the mixing space (inlet velocity). The inventors of the present invention believe that, as a result, a vortex or rotating flow of the fluids is generated in the mixing space and this flow enables the first fluid and the second fluid to be efficiently mixed.

From the standpoint that such a vortex or rotating flow is easily generated, a distance between the outlet portion of the first microtubular flow path and an outlet portion of the mixing space through which a mixture of the first and second fluids flows and between the outlet portion of the second microtubular flow path and the outlet portion of the mixing space is preferably 2 to 100 mm, and more preferably 10 to 200 mm. Note that the term "outlet portion of the mixing space" in the present invention refers to the opening of the mixture, the opening being denoted by 5B in Fig. 1.

The first fluid (F1) and the second fluid (F2) are respectively supplied from the fluid supply units 1A and 2A into the case C and respectively flow through the first microtubular flow path and the second microtubular flow path formed in the stacked body 110. The first fluid (F1) reaching the outlet of the first microtubular flow path and the second fluid (F2) reaching the outlet of the second microtubular flow path are then discharged to the mixing space S3 that is connected to the outlet portions of the microtubular flow paths and mixed. The resulting fluid mixture (F3) is sent from the sending unit 5A to the outside of the case C. The positions etc. of the case C, the fluid supply units 1A and 2A, and the sending unit 5A of the micromixer 1 are not limited to the above structure, and may be appropriately changed.

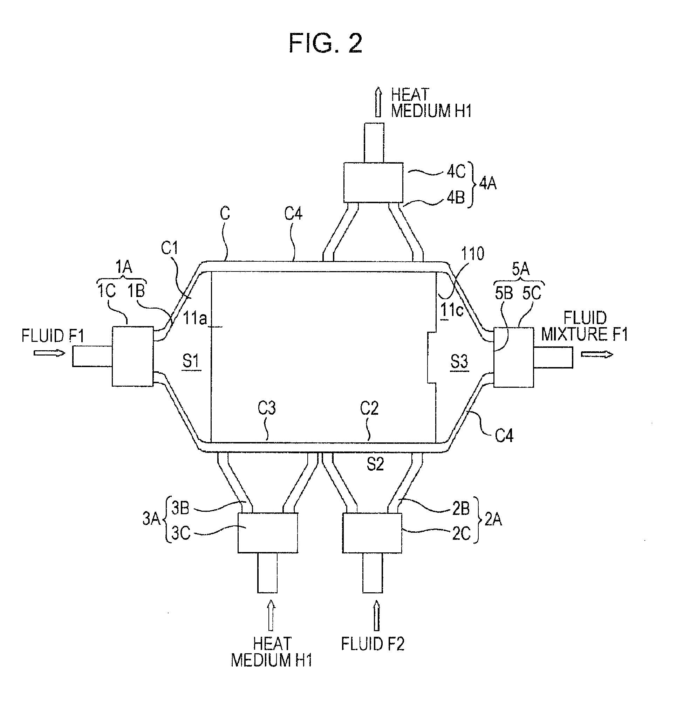

A micromixer 2 illustrated in Fig. 2 and including a mixing space having a different shape can also be exemplified as a micromixer according to another embodiment. In Fig. 2, an outlet portion of a first microtubular flow path and an outlet portion of a second microtubular flow path, the outlet portions being in contact with a side face 11c of a stacked body 110, are located on a surface recessed from the side face 11c. This recess functions as a part of the mixing space.

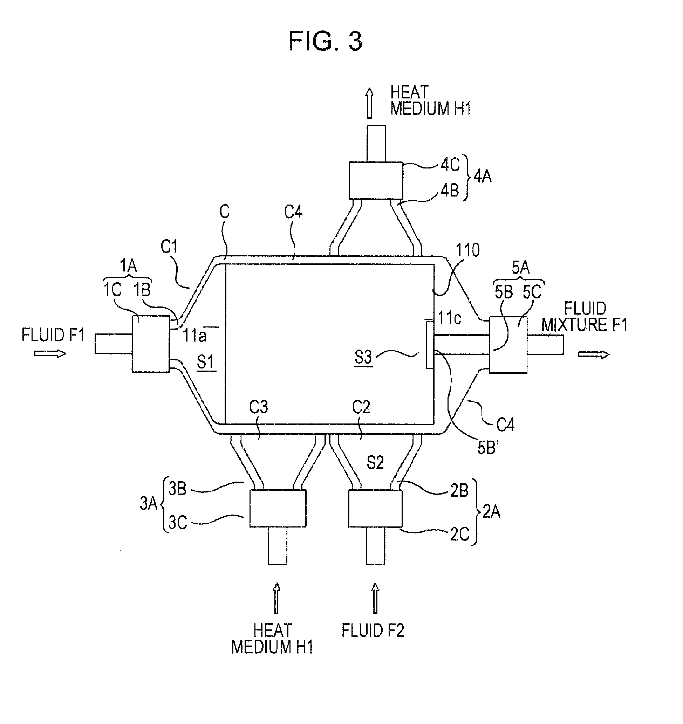

A micromixer 3 illustrated in Fig. 3 can also be exemplified as a micromixer according to another embodiment. As in the micromixer 2, an outlet portion of a first microtubular flow path and an outlet portion of a second microtubular flow path of the micromixer 3, the outlet portions being in contact with a side face 11c of a stacked body 110, are located on a surface recessed from the side face 11c. A pipe for discharging the resulting fluid mixture to the outside of the micromixer is provided on the recessed portion. Accordingly, unlike the micromixer 2, only the recessed portion functions as a mixing space in the micromixer 3. In this case, the term "outlet portion of the mixing space" refers to the opening of the mixture, the opening being denoted by 5B' in Fig. 3.

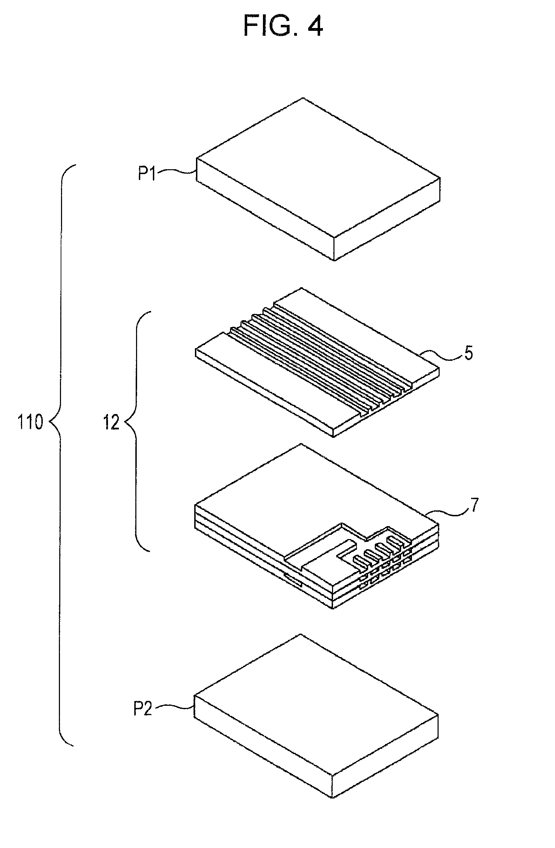

Next, the stacked body 110 will be described. As illustrated in Fig. 4, the stacked body 110 includes rectangular cover plates P1 and P2 and a group 12 of plates each having a flow path thereon, the group 12 of plates being arranged between the cover plates P1 and P2.

The group 12 of plates is constituted by stacking two first plates 5 and two second plates 7. In this embodiment, the first plates and the two second plates are alternately stacked to form a stacked body.

The cover plates P1 and P2, the first plates 5, and the second plates 7 are formed so that the outer shapes thereof are the same rectangular shape. The materials of the cover plates P1 and P2, the first plates 5, and the second plates 7 are not particularly limited. Examples of the materials include metallic materials, resins, glass, and ceramics. The materials are not particularly limited as long as a process for forming a flow path can be easily performed and the plates can be fixed to each other in a close contact state in which the leakage of a liquid etc. do not readily occur. The plates may be composed of the same material or different materials. For example, the plates may each be composed of a stainless steel and may be fixed in a close contact state by diffusion bonding. A method for processing the plate can be suitably selected from known methods such as injection molding, a solvent cast method, a melt replica method, cutting, etching, photolithography, and laser ablation in accordance with the material.

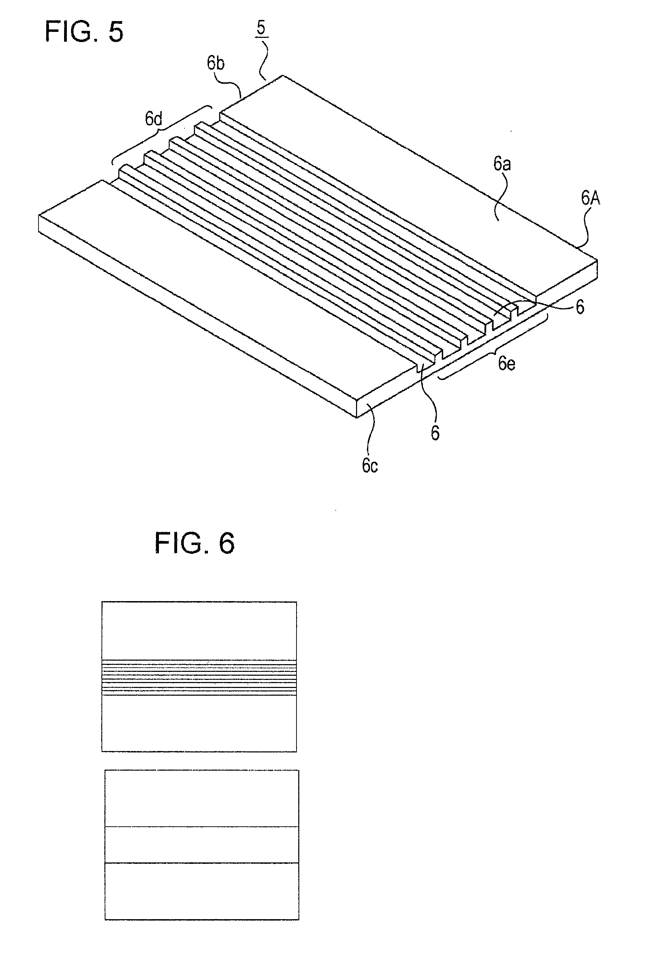

Next, the first plate 5 and the second plate 7 will be described in detail. As illustrated in Fig. 5, the first plate 5 includes a rectangular, plate-shaped first-microtubular-flow-path-forming portion 6A.

The first-microtubular-flow-path-forming portion 6A has at least one first microtubular flow path 6 in a central part in the short direction (Y direction in the figure) in a top surface 6a thereof. The first microtubular flow path 6 has a groove shape and extends from a left-side end 6b to a right-side end 6c of the first-microtubular-flow-path-forming portion 6A. The first microtubular flow path 6 is open at the left-side end 6b, the right-side end 6c, and the top surface 6a. The opening on the left-side end 6b functions as an inlet 6d of the first microtubular flow path 6, and the opening on the right-side end 5c functions as an outlet 6e of the first microtubular flow path 6. The inlet 6d is connected to the first fluid supply unit 1A to which the first fluid F1 is supplied.

The first microtubular flow path 6 is a flow path whose cross section in a direction orthogonal to the flowing direction has a rectangular shape, and extends from the left-side end 6b to the right-side end 6c. In order to ensure the uniformity of the temperature distribution of the fluid and the strength of the device, the first microtubular flow path 6 preferably has, for example, a width in the range of 0.1 mm or more and 100 mm or less and a depth in the range of 0.1 mm or more and 5 mm or less, and more preferably, a width in the range of 0.1 mm or more and 20 mm or less and a depth in the range of 0.1 mm or more and 2 mm or less. That is, the shape of a large diameter portion 16 may be any shape of a flow path as long as the pressure loss
(loss of pressure) does not become excessively high, clogging of the flow path does not readily occur, heating and cooling of the flow path can be quickly controlled, and productivity can be improved. The pressure loss is preferably 5 MPa or less, and more preferably 1 MPa or less.

The cross-sectional area of the fluid that liquid-tightly flows through the first microtubular flow path 6 is preferably 0.01 to 500 mm², and more preferably 0.01 to 40 mm².

In Fig. 4, five first microtubular flow paths 6 are arranged, but the number of the first microtubular flow paths 6 is not particularly limited. In the case where a plurality of first microtubular flow paths 6 are arranged, the widths and the depths of the microtubular flow paths 6 may be the same or different. The flow path width of the inlet of a first microtubular flow path 6 and the flow path width of the outlet of the first microtubular flow path 6 may be the same or different. Fig. 6 illustrates other examples of the first plate.

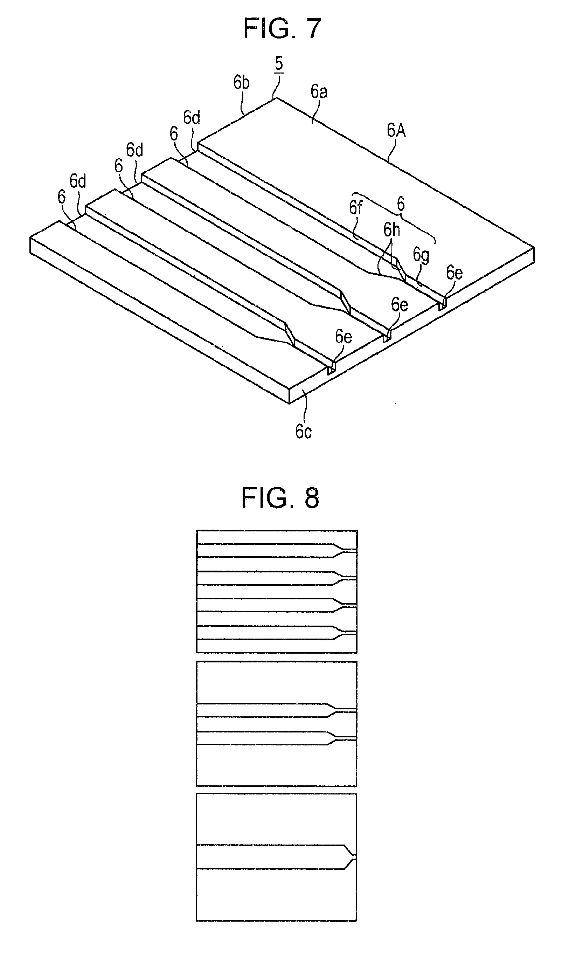

In the first plate described above, a cross-sectional area of the microtubular flow path in the outlet portion connected to the mixing space is preferably smaller than a cross-sectional area of the microtubular flow path in the inlet portion connected to the fluid supply path from the standpoint of obtaining a micromixer having a good mixing efficiency. A first plate of this embodiment is illustrated in Fig. 5. The plate will now be described with reference to Fig. 7.

In Fig. 7, a first microtubular flow path 6 includes a large diameter portion 6f having a large flow path diameter, a small diameter portion 6g having a small flow path diameter, and a tapered portion 6h that makes the change in the diameter from the large diameter portion 6f to the small diameter portion 6g gentle.

The large diameter portion 6f is a flow path whose cross section in a direction orthogonal to the flowing direction has a rectangular shape, and extends from a left-side end 6b toward the front of a right-side end 6c. In order to ensure the uniformity of the temperature distribution of a fluid and the strength of the device, the large diameter portion 6f preferably has, for example, a width in the range of 0.1 mm or more and 100 mm or less and a depth of 5 mm or less, and more preferably, a width in the range of 0.1 mm or more and 20 mm or less and a depth of 2 mm or less. That is, the shape of the large diameter portion 16 may be any shape of a flow path as long as the pressure loss does not become excessively high, clogging of the flow path does not readily occur, heating and cooling of the flow path can be quickly controlled, and productivity can be improved. The pressure loss is preferably 5 MPa or less, and more preferably 1 MPa or less.

The large diameter portion 6f is a flow path whose cross section in a direction orthogonal to the flowing direction has a rectangular shape, and extends from the left-side end 6b toward the front of the right-side end 6c. In order to ensure the uniformity of the temperature distribution of a fluid and the strength of the device, the large diameter portion 6f preferably has, for example, a width in the range of 0.1 mm or more and 100 mm or less and a depth in the range of 0.1 mm or more and 5 mm or less, and more preferably, a width in the range of 0.1 mm or more and 20 mm or less and a depth in the range of 0.1 mm or more and 2 mm or less. Still more preferably, the width is in the range of 0.1 mm or more and 20 mm or less, and the depth is in the range of 0.1 mm or more and 1 mm or less. That is, the shape of the large diameter portion 16 may be any shape of a flow path as long as the pressure loss does not become excessively high, clogging of the flow path does not readily occur, heating and cooling of the flow path can be quickly controlled, the flow rate of the fluid can be increased, a shear stress is applied, and molecular diffusion occurs in a state involving a slightly turbulent flow to improve the mixing efficiency. The pressure loss is preferably 5 MPa or less, and more preferably 1 MPa or less.

The cross-sectional area of the first microtubular flow path 6f is preferably 0.01 to 500 mm², more preferably 0.01 to 40 mm², and still more preferably 0.01 to 2.0 mm².

The small diameter portion 6g is also a flow path formed so as to have a rectangular cross section and extends from the front of the right-side end 6f to the right-side end 6c. It is sufficient that the small diameter portion 6g has a cross sectional area at least smaller than the cross-sectional area of the large diameter portion 16. The small diameter portion 6g preferably has, for example, a width in the range of 0.1 mm or more and 20 mm or less and a depth in the range of 0.1 mm or more and 5 mm or less, and more preferably, a width in the range of 0.1 mm or more and 5 mm or less and a depth in the range of 0.1 mm or more and 2 mm or less. Still more preferably, the width is in the range of 0.1 mm or more and 5 mm or less, and the depth is in the range of 0.1 mm or more and 1 mm or less. That is, the shape of the small diameter portion 17 may be any shape of a flow path as long as the pressure loss does not become excessively high, clogging of the flow path does not readily occur, heating and cooling of the flow path can be quickly controlled, the flow rate of the fluid can be increased, a shear stress is applied, and molecular diffusion occurs in a state involving a slightly turbulent flow to improve the mixing efficiency.

The cross-sectional area of the first microtubular flow path 6g is preferably 0.01 to 100 mm², more preferably 0.01 to 10 mm², and still more preferably 0.01 to 5 mm².

The rate of the fluid when the fluid flows through the first microtubular flow path 6g is preferably 0.5 m/sec or more, more preferably 1.0 m/sec or more, still more preferably 3.0 m/sec or more, and particularly preferably 5.0 m/sec or more because a shear stress applied to the fluid increases. In particular, in the case where high-viscosity fluids are mixed with each other or fluids having significantly different viscosities are mixed with each other, the flow rate is preferably 1.0 m/sec or more.

The first microtubular flow path 6 is a flow path whose cross section in a direction orthogonal to the flowing direction has a rectangular shape, and extends from the left-side end 6b to the right-side end 6c. In order to ensure the uniformity of the temperature distribution of the fluid and the strength of the device, the first microtubular flow path 6 preferably has, for example, a width in the range of 0.1 mm or more and 100 mm or less and a depth in the range of 0.1 mm or more and 5 mm or less, more preferably, a width in the range of 0.1 mm or more and 10 mm or less and a depth in the range of 0.1 mm or more and 1 mm or less. More preferably, the width is in the range of 0.1 mm or more and 20 mm or less, and the depth is in the range of 0.1 mm or more and 2 mm or less. Still more preferably, the width is in the range of 0.1 mm or more and 2 mm or less, and the depth is in the range of 0.1 mm or more and 0.5 mm or less. That is, the shape of the large diameter portion 16 may be any shape of a flow path as long as the pressure loss does not become excessively high, clogging of the flow path does not readily occur, heating and cooling of the flow path can be quickly controlled, the flow rate of the fluid can be increased, a shear stress is applied, and molecular diffusion occurs in a state involving a slightly turbulent flow to improve the mixing efficiency. The pressure loss is preferably 5 MPa or less, and more preferably 1 MPa or less.

In Fig. 7, three first microtubular flow paths 6 are arranged, but the number of the first microtubular flow paths 6 is not particularly limited. In the case where a plurality of first microtubular flow paths 6 are arranged, the widths and the depths of the first microtubular flow paths 6 may be the same or different. The flow path width of the inlet of a first microtubular flow path 6 and the flow path width of the outlet of the first microtubular flow path 6 may be the same or different. Fig. 8 illustrates other examples of the first plate.

The viscosity of the fluid that flows through the first microtubular flow path is preferably 3,000 mPa·s or less, more preferably 1,000 mPa·s or less, and still more preferably 500 mPa·s or less.
The viscosity of the fluid is particularly preferably 100 mPa·s or less.

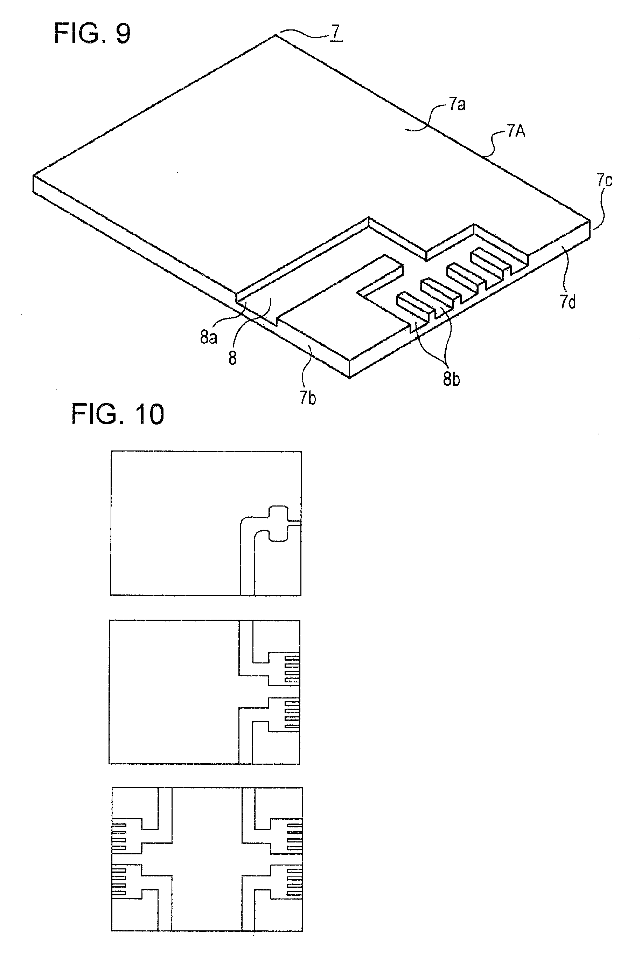

Next, the second plate 7 will be described in detail. As illustrated in Fig. 9, the second plate 7 includes a rectangular, plate-shaped second-microtubular-flow-path-forming portion 7A.

The second-microtubular-flow-path-forming portion 7A has a single second microtubular flow path 8 in a top surface 7a thereof. The second microtubular flow path 8 has a groove shape and extends from a lower-side end 7b of the second-microtubular-flow-path-forming portion 7A in the short direction (7c direction, Y direction in the figure). Furthermore, the second microtubular flow path 8 is once bent near the center of the Y direction at a right angle in the direction of the right end, and is open at the lower-side end 7b, a right-side end 7d, and the top surface 7a. The opening on the lower-side end 7b functions as an inlet 8a of the second microtubular flow path 8, and the opening on the right-side end 7d functions as an outlet 8b of the first microtubular flow path 6. The inlet 8a is connected to the second fluid supply unit 2A to which the second fluid F1 is supplied.

According to the micromixer of the present invention, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path of the inlet portion (in this description, this plate is described as the second plate). With this structure, the second fluid F2 flows through the inlet 8a of the large diameter portion in the inlet portion and flows into the small diameter portions 8b. The second fluid F2 flown into each of the small diameter portions flows into the outlet 8b at a flow rate higher than the flow rate when the second fluid F2 flows into the inlet, and flows into the mixing space (S3 in Fig. 1). As a result, the mixing rate of the first fluid F1 and the second fluid F2 can be increased. In particular, this effect can be achieved in the case where at least one of the first fluid F1 and the second fluid F2 is a fluid that is not easily mixed due to a low fluidity thereof, i.e., a high-viscosity fluid, or in the case where the first fluid F1 and the second fluid F2 have significantly different viscosities.

In order to ensure the uniformity of the temperature distribution of the fluid and the strength of the device, the large diameter portion preferably has, for example, a width in the range of 0.1 mm or more and 100 mm or less and a depth in the range of 0.1 mm or more and 5 mm or less, and more preferably, a width in the range of 0.1 mm or more and 20 mm or less and a depth in the range of 0.1 mm or more 2 mm or less. That is, the shape of the large diameter portion may be any shape of a flow path as long as the pressure loss does not become excessively high, clogging of the flow path does not readily occur, heating and cooling of the flow path can be quickly controlled, and productivity can be improved. The pressure loss is preferably 5 MPa or less, and more preferably 1 MPa or less.

The cross-sectional area of the fluid that liquid-tightly flows through the large diameter portion is preferably 0.01 to 500 mm², and more preferably 0.01 to 40 mm².

It is sufficient that the cross sectional area of the small diameter portion is at least smaller than the cross-sectional area of the large diameter portion. The small diameter portion preferably has, for example, a width in the range of 0.1 mm or more and 20 mm or less and a depth in the range of 5 mm or less, and more preferably, a width in the range of 0.1 mm or more and 5 mm or less and a depth in the range of 0.1 mm or more and 2 mm or less. That is, the small diameter portion preferably has a shape of a flow path in which the pressure loss does not become excessively high, clogging of the flow path does not readily occur, heating and cooling of the flow path can be quickly controlled, and productivity can be improved. The pressure loss is preferably 5 MPa or less, and more preferably 1 MPa or less.

The cross-sectional area of the fluid that liquid-tightly flows through the small diameter portion is preferably 0.01 to 100 mm², and more preferably 0.01 to 10 mm².

The rate of the fluid when the fluid flows through the second microtubular flow path 8b is preferably 0.5 m/sec or more, more preferably 1.0 m/sec or more, still more preferably 3.0 m/sec or more, and particularly preferably 5.0 m/sec or more because a shear stress applied to the fluid increases. In particular, in the case where high-viscosity fluids are mixed with each other or fluids having significantly different viscosities are mixed with each other, the flow rate is preferably 1.0 m/sec or more.

The viscosity of the fluid that flows through the second microtubular flow path is preferably 3,000 mPa·s or less, more preferably 1,000 mPa·s or less, and still more preferably 500 mPa·s or less. The viscosity of the fluid is particularly preferably 100 mPa·s or less.

As illustrated in Fig. 9, in the second plate, the outlet portion connected to the mixing space is divided by a plurality of walls arranged in parallel with a direction of the flow of the fluid, thus forming a plurality of flow paths. Alternatively, as illustrated in Fig. 10, a single flow path may be provided. The number of walls is, for example, 1 to 250, and more preferably 1 to 50. The number of second microtubular flow paths formed in the second plate may be one, as illustrated in Fig. 9. Alternatively, as illustrated in Fig. 10, the number of second microtubular flow paths may be two or more.

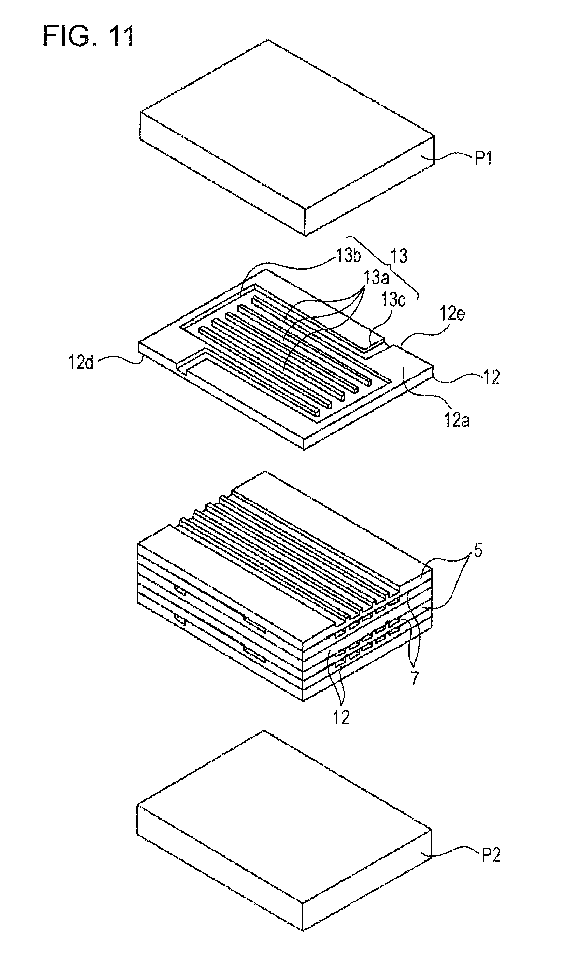

The micromixer of the present invention may include a temperature control plate through which a medium for heat exchange flows. Fig. 11 illustrates an example of a micromixer in which temperature control plates, first plates, and second plates are stacked.

As illustrated in Fig. 11, a temperature control plate 12 includes a temperature control flow path 13 on a surface 12a thereof. The temperature control flow path 13 has a cross section having a recessed groove shape and is arranged with predetermined intervals. The cross-sectional area of the temperature control flow path 12 is not particularly limited as long as heat can be transmitted to a reaction flow path, and is in the range of about 0.02 to 500.0 (mm²). The cross-sectional area is more preferably in the range of about 0.05 to 40.0 (mm²). The number of temperature control flow paths 6 can be appropriately determined in consideration of heat exchange efficiency and is not particularly limited. The number of temperature control flow paths is, for example, 1 to 1,000, and preferably 1 to 100 per plate. For example, the flow path preferably has a width in the range of 0.2 mm or more and 100 mm or less and a depth in the range of 0.1 mm or more and 5 mm or less, and more preferably, a width in the range of 0.5 mm or more and 20 mm or less and a depth in the range of 0.1 mm or more and 2 mm or less. That is, the shape of the large diameter portion 16 may be any shape of a flow path as long as the pressure loss does not become excessively high, clogging of the flow path does not readily occur, heating and cooling of the flow path can be quickly controlled, and productivity can be improved.

As illustrated in Fig. 11, the temperature control flow path 12 may include a plurality of main flow paths 13a arranged in the longitudinal direction of the temperature control plate 12, and a supply-side flow path 13b and a discharge-side flow path 13c that are connected to the main flow paths 13a at an end on the upstream side of the main flow paths 13a and at an end on the downstream side of the main flow paths 13a, respectively.

In Fig. 11, the supply-side flow path 13b and the discharge-side flow path 13c are bent twice at a right angle and respectively open from side faces 12d and 12e of the temperature control plate to the outside. Regarding the number of flow paths of the temperature control flow path 12, only the number of the main flow paths 13a of the temperature control flow path 12 is two or more, the number of the supply-side flow paths 13b is one, and the number of the discharge-side flow paths 13c is one.

In the stacked body 110 having the structure described above, the first fluid (F1) supplied from the first fluid supply unit 1A into the case C in a pressurized state is temporarily stored in the storage portion S1 and is then divided into the plurality of first microtubular flow paths 6 provided in the stacked body 110. The second fluid (F2) supplied from the second fluid supply unit 2A into the case C in a pressurized state is temporarily stored in the storage portion S2 and is then divided into the plurality of second microtubular flow paths 8 provided in the stacked body 110.

The first fluid (F1) flown into the first microtubular flow paths 6 of the first plate 5 flows to the outlet 6e of the first microtubular flow paths 6, and is sent to the mixing space S3. The second fluid (F2) flown into the second microtubular flow paths 8 of the second plate 7 is sent from a large diameter portion 9 to a small diameter portion 10 while increasing the flow rate, and is sent from the outlet 8b to the mixing space S3.

The second fluid (F2) sent to the mixing space S3 with an increase in the flow rate is mixed with the second fluid (F2) sent to the mixing space S3. Since the rate of the second fluid (F2) is increased at this time, the mixing efficiency in the mixing space S3 is improved.

The first fluid (F1) and the second fluid (F2) are mixed with each other while generating a turbulent flow in the mixing space S3. The resulting fluid mixture (F3) flows toward the sending unit 5A of the fluid mixture. The fluid mixture (F3) is sent from the sending unit 5A toward the outside of the case C.

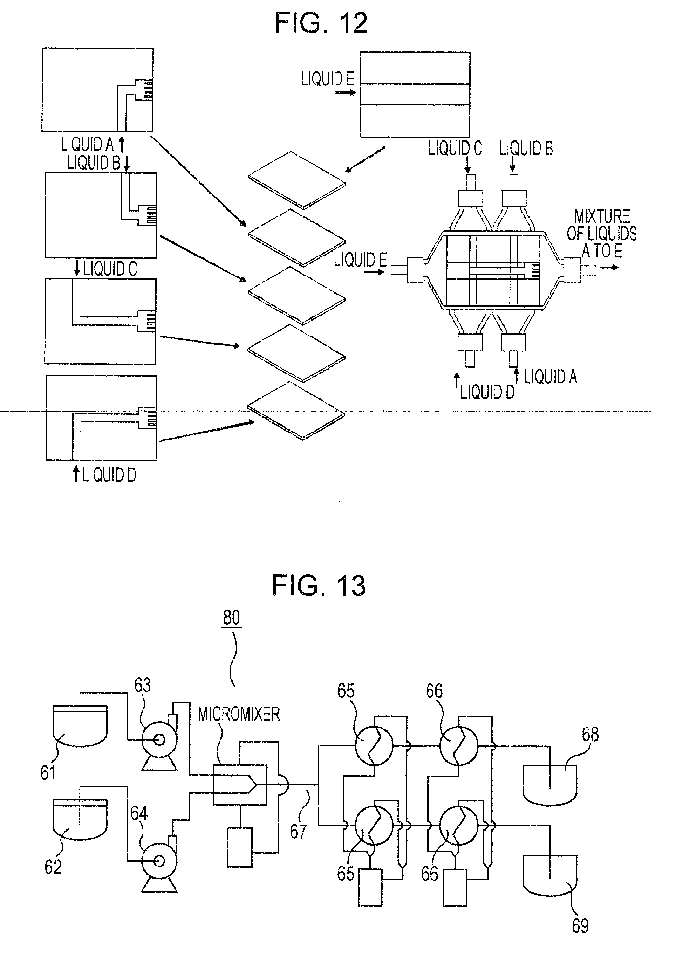

According to the micromixer of the present invention, since the number of inlet portions of the microtubular flow paths of the second plate can be easily increased, a fluid mixture of various types of fluids can also be obtained. In the case where various types of fluids flow through the micromixer of the present invention, for example, a preferred example of a micromixer includes a stack unit in which at least one first plate and at least two second plates are stacked, in which an inlet portion of a microtubular flow path of the first plate is disposed on a side face other than side faces on which the inlet portions of the microtubular flow paths of the second plates are disposed. Specifically, as illustrated in Fig. 12, the micromixer may be a micromixer for mixing five types of solutions, the micromixer including a first plate through which a liquid E flows and four second plates through which a liquid A, a liquid B, a liquid C, and a liquid D flow, respectively, the first plate and the second plates being stacked.

A method for producing a liquid mixture of the present invention uses the micromixer of the present invention and includes allowing the first fluid and the second fluid that have a viscosity ratio of 10 to 3,000 to respectively flow through the first microtubular flow path and the second microtubular flow path to obtain a fluid mixture containing the first fluid and the second fluid in a mixing space. When fluids having significantly different viscosities are mixed using an existing mixer, a pressure loss, clogging of a fluid, etc. occur, resulting in a difficulty in flowing, and the fluids cannot be sufficiently mixed. According to the production method of the present invention, even in the case where fluids having significantly different viscosities, namely, a viscosity ratio of 10 to 3,000, are mixed with each other, a low-viscosity fluid does not intermittently flow but stably flows and the fluids can be satisfactorily mixed without causing the above problem by using the micromixer of the present invention.

In the production method, out of a first fluid and a second fluid, a fluid having a higher viscosity is preferably supplied to the microtubular flow path which has an inlet portion connected to the fluid supply path and constituted by a single flow path and in which a cross-sectional area in an outlet portion connected to the mixing space is smaller than a cross-sectional area in the inlet portion because the first fluid and the second fluid can be uniformly mixed. A plate having such a flow path is disclosed as the second plate in the present invention.

Examples of combinations in which the viscosities of fluids are significantly different preferably include the following:
1. A combination for obtaining a urethane (meth)acrylate using a compound (A) having an isocyanate group and a (meth)acrylate (A) having a hydroxyl group.
2. A combination for obtaining an epoxy (meth)acrylate using a (meth)acrylate having a hydroxy group and a compound having an epoxy group.
3. A combination for obtaining a polyurethane resin using a compound having active hydrogen and a compound having an isocyanate group.
4. A combination for obtaining an alkyl substituted benzene diol using a solution containing an alkyl phenol and a solution containing hydrogen peroxide.
5. A combination for obtaining pigment fine particles having an anthraquinone structure using water and a sulfuric acid solution of a pigment having an anthraquinone structure.

A method for producing a urethane (meth)acrylate and a method for producing pigment fine particles having an anthraquinone structure using the micromixer of the present invention will now be described as examples of preferred combinations.

### <Method for producing urethane (meth)acrylate>

A method for producing a urethane (meth)acrylate of the present invention uses a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion.
This method includes a first step of allowing a compound (A) having an isocyanate group to flow through one of the first microtubular flow path and the second microtubular flow path and allowing a (meth)acrylate (B) having a hydroxyl group to flow through the other microtubular flow path to obtain a fluid mixture containing the compound (A) and the compound (B) in the mixing space, and
a second step of allowing the compound (A) and the compound (B) in the fluid mixture to react with each other.

The method for producing a urethane (meth)acrylate of the present invention preferably uses the micromixer of the present invention in which, in the second plate, the microtubular flow path has an inlet portion connected to the fluid supply path and constituted by a single flow path and an outlet portion connected to the mixing space, and a cross-sectional area of the fluid that liquid-tightly flows through the microtubular flow path in the outlet portion is smaller than a cross-sectional area of the fluid that liquid-tightly flows through the single microtubular flow path in the inlet portion,
and preferably includes a first step of allowing a compound (A) having an isocyanate group to flow through the first microtubular flow path of the first plate and allowing a (meth)acrylate (B) having a hydroxyl group to flow through the second microtubular flow path of the second plate to obtain a fluid mixture containing the compound (A) and the compound (B) in the mixing space; and
a second step of allowing the compound (A) and the compound (B) in the fluid mixture to react with each other This method is preferable from the standpoint that the compound (A) and the compound (B) are satisfactorily mixed, and a urethane (meth)acrylate having a stable quality can be produced.

In general, a (meth)acrylate having a hydroxyl group has a viscosity that is about 100 times higher than that of the compound (A) having an isocyanate group. In the present invention, by allowing the compound (B) having a high viscosity to flow through the second microtubular flow path, the compound (B) rapidly flows through the flow path, and thus a mixture in which the compound (A) and the compound (B) are uniformly mixed can be obtained in the mixing space.

In the first step of the present invention, a fluid mixture containing the compound (A) and the compound (B) is obtained in the mixing space of the micromixer. Examples of the compound (A) include isocyanate compounds such as aromatic isocyanate compounds, aliphatic isocyanate compounds, and alicyclic isocyanate compounds. Specific examples thereof include tolylene diisocyanate, diphenylmethane diisocyanate, hydrogenated diphenylmethane diisocyanate, phenylmethane polyisocyanate, modified diphenylmethane diisocyanate, xylylene diisocyanate, hydrogenated xylylene diisocyanate, hexamethylene diisocyanate, trimethylhexamethylene diisocyanate, tetramethylxylylene diisocyanate, isophorone diisocyanate, norbornene diisocyanate, phenylene diisocyanate, lysine diisocyanate, lysine triisocyanate, naphthalene diisocyanate, 2-(meth)acryloyloxyethyl isocyanate, 1,1-bis(acryloylmethyl)ethyl isocyanate, phenyl isocyanate, para-toluenesulfonyl isocyanate, octadecyl isocyanate, and butyl isocyanate; trimers and multimers of any of these isocyanates, adduct-type isocyanate, compounds, biuret-type isocyanate compounds, allophanate-type isocyanate compounds, urethodione-type isocyanate compounds of any of these isocyanates, and blocked products thereof; and reaction products of any of these isocyanates and a polyol. These compounds may be used alone or in combination of two or more compounds.

Examples of the compound (B) include hydroxyl group-containing (meth)acrylate compounds having one (meth)acryloyl group, such as 2-hydroxyethyl (meth)acrylate, 2-hydroxypropyl (meth)acrylate, 2-hydroxybutyl (meth)acrylate, 4-hydroxybutyl (meth)acrylate, 2-hydroxyethylacryloyl phosphate, 2-(meth)acryloyloxyethyl-2-hydroxypropyl phthalate, and caprolactone-modified 2-hydroxyethyl (meth)acrylate; and

hydroxyl group-containing (meth)acrylate compounds having two or more (meth)acryloyl groups, such as 2-hydroxy-3-(meth)acryloyloxypropyl (meth)acrylate, pentaerythritol tri(meth)acrylate, dipentaerythritol penta(meth)acrylate, caprolactone-modified dipentaerythritol penta(meth)acrylate, and ethylene oxide-modified pentaerythritol tri(meth)acrylate. These compounds may be used alone or in combination of two or more compounds.

A fluid mixture of the compound (A) and the compound (B) is obtained, for example, as follows: A tank for containing the compound (A) is connected to the second microtubular flow path of the micromixer, and a tank for containing the compound (B) is connected to the first microtubular flow path of the micromixer. The compounds are allowed to flow through the microtubular flow paths at a flow rate of about 100 g/min using pumps, and mixed in the mixing space.

When the compound (A) and compound (B) are allowed to follow through the microtubular flow paths, the compounds may be heated to, for example, about 60°C to decrease the viscosity.

The fluid mixture discharged from the mixing space is heated to allow the compound (A) and the compound (B) to react with each other. For example, in the case where the reaction is performed by a batch process, the reaction may be performed at a temperature of 70°C to 100°C. In the case where the reaction is continuously performed using a reactor such as a tube reactor, the reaction may be performed at a temperature of 100°C to 180°C. Although the reaction is performed at a high temperature when the reaction is continuously performed using a reactor, runaway of the reaction can be prevented by cooling the reaction product immediately after the completion of the reaction.

### <Method for producing pigment fine particles having anthraquinone structure>

A method for producing pigment fine particles having an anthraquinone structure of the present invention uses a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion.
This method includes a first step of mixing water (C) and a sulfuric acid solution (D) of a pigment having an anthraquinone structure in the mixing space by allowing the water (C) to flow through one of the first microtubular flow path and the second microtubular flow path and allowing the sulfuric acid solution (D) to flow through the other microtubular flow path to obtain a fluid mixture in which the pigment having an anthraquinone structure is deposited, and
a second step of cooling the fluid mixture in which the pigment is deposited.

The method for producing pigment fine particles having an anthraquinone structure of the present invention preferably uses the micromixer of the present invention in which, in the second plate, the microtubular flow path has an inlet portion connected to the fluid supply path and constituted by a single flow path and an outlet portion connected to the mixing space, and a cross-sectional area of the fluid that liquid-tightly flows through the microtubular flow path in the outlet portion is smaller than a cross-sectional area of the fluid that liquid-tightly flows through the single microtubular flow path in the inlet portion,
and preferably includes a first step of mixing water (C) and a sulfuric acid solution (D) of a pigment having an anthraquinone structure in the mixing space by allowing the water (C) to flow through the first microtubular flow path of the first plate and allowing the sulfuric acid solution (D) to flow through the second microtubular flow path of the second plate to obtain a fluid mixture in which the pigment having an anthraquinone structure is deposited; and
a second step of cooling the fluid mixture in which the pigment is deposited. This method is preferable from the standpoint that the water (C) and the sulfuric acid solution (D) can be efficiently mixed and pigment fine particles having a small average particle diameter can be obtained.

The sulfuric acid solution (D) of a pigment having an anthraquinone structure has a viscosity that is about 30 times higher than that of water (C). In the present invention, by allowing the sulfuric acid solution (D) having a high viscosity to flow through the second microtubular flow path, the sulfuric acid solution (D) rapidly flows through the flow path, and thus a mixture in which the compound (A) and the compound (B) are uniformly mixed can be obtained in the mixing space.

In the first step of the present invention, water (C) and the sulfuric acid solution (D) are mixed in the mixing space of the micromixer to obtain a fluid mixture in which a pigment having an anthraquinone structure is deposited by crystallization. Examples of the pigment having an anthraquinone structure for preparing the sulfuric acid solution (D) include C.I. Pigment Red 83, C.I. Pigment Red 177, and C.I. Pigment Red 89. Among these, C.I. Pigment Red 177 is preferable because pigment fine particles having a small particle diameter are obtained and can be suitably used as a red filter segment forming a color filter.

The concentration of sulfuric acid used for preparing a sulfuric acid solution (viscosity at room temperature: 27 mPa·s) in which a pigment having an anthraquinone structure is dissolved in the sulfuric acid is not particularly limited as long as the pigment having an anthraquinone structure is dissolved and does not undergo a chemical reaction such as sulfonation or oxidation, but is usually in the range of 70% to 100% by weight. In particular, use of a commercially available concentrated sulfuric acid having a concentration of 95% to 98% by weight is advantageous economically and in terms of and handling.

The content of the pigment having an anthraquinone structure in the sulfuric acid solution is 2% to 25% by mass. A content of less than 2% by mass is not preferable because it is difficult to realize both good production efficiency and a good quality of the pigment having a particle diameter on the order of nanometers. A content of more than 25% by mass is not preferable because redeposition of the pigment is caused by the effect of moisture in the air or the like and it is difficult to obtain the pigment having a particle diameter on the order of nanometers. The content of the pigment having an anthraquinone structure in the sulfuric acid solution is preferably 3% to 20% by mass, and more preferably 4% to 15% by mass.

In preparation of the sulfuric acid solution, in order to prevent a chemical reaction, such as sulfonation or oxidation, of the pigment having an anthraquinone structure from occurring, the pigment having an anthraquinone structure and sulfuric acid are preferably mixed so that the temperature of the sulfuric acid solution becomes 45°C or lower, and more preferably in the range of 32°C to 42°C.

A fluid mixture in which a pigment having an anthraquinone structure is deposited is obtained, for example, as follows: A tank for containing the sulfuric acid solution (D) is connected to the second microtubular flow path of the micromixer, and a tank for containing the water (C) is connected to the first microtubular flow path of the micromixer. The fluids are allowed to flow through the microtubular flow paths using pumps, and mixed in the mixing space. In the first step, 300 to 3,000 parts by mass of the water (C) is preferably mixed with 100 parts by mass of the sulfuric acid solution (D) because pigment fine particles having a small particle diameter are obtained, and production efficiency is also good. Regarding the mixing ratio of the sulfuric acid solution (D) to the water (C), the water (C) is preferably 500 to 1,500, and more preferably 700 to 1,200 relative to 100 parts by mass of the sulfuric acid solution (D).

The liquid temperature of the fluid mixture obtained in the first step is preferably maintained at a temperature at which a solution state of the pigment is maintained and at 50°C or lower because pigment fine particles having a small particle diameter are easily obtained. The liquid temperature is more preferably 5°C to 40°C, and still more preferably 10°C to 30°C.

The temperature of the water (C) used in the first step is preferably 1°C to 45°C because a fluid mixture having a liquid temperature of 50°C or lower is easily obtained.

The fluid mixture obtained in the first step is cooled in the second step. In the cooling, by adjusting the temperature of the fluid mixture to 30°C or lower and to a temperature at which a solution state is maintained, pigment fine particles having a small particle diameter can be obtained. In the case where the temperature of the fluid mixture is adjusted to 30°C or lower, the adjustment is preferably performed within 30 seconds from the time when the fluid mixture in which a pigment is deposited is obtained in the first step.

The first step is performed in the mixing space of the micromixer of the present invention, and the resulting fluid is then discharged from the mixing space. Thus, the second step is performed outside the micromixer of the present invention. The second step may be performed by, for example, a batch process. Alternatively, in the second step, the cooling may be continuously performed using a reactor such as a tube reactor.

After the completion of the second step, a dispersion in which pigment fine particles are dispersed in a solution is obtained. This dispersion may be used as a final product. Alternatively, the fine particles may be collected by any means, and may be used as a dry pigment.

In the case where the pigment fine particles obtained by the production method of the present invention are used as a colorant, the prepared pigment fine particles may be used without further treatment or may be treated with a surfactant, a resin, rosin, a pigment derivative, or the like and then used.

According to the embodiments described above, the micromixer of the present invention can achieve the following advantage.
(1) In the embodiments described above, the micromixer 1 includes a stack unit in which a second plate is stacked on a first plate having a first microtubular flow path, and a mixing space. Furthermore, in at least one of the first plate and the second plate, the microtubular flow path has an inlet portion constituted by a single flow path, and a cross-sectional area of a fluid that liquid-tightly flows through the microtubular flow path in an outlet portion is smaller than a cross-sectional area of the fluid that liquid-tightly flows through the single microtubular flow path in the inlet portion. With this structure, the mixing efficiency of the first fluid and the second fluid can be enhanced. Even in the case where high-viscosity fluids are mixed with each other or different-viscosity fluids having different viscosities are mixed with each other, a low-viscosity fluid does not intermittently flow but stably flows and the mixing can be efficiently performed.

### EXAMPLES

The present invention will now be described in more detail by way of Examples. In Examples, "%" is on a weight basis unless otherwise specified.

### <Reaction device used in Example 1>

A reaction device illustrated in Fig. 13 was used in Example 1. In this device, the micromixer 1 illustrated in Fig. 1 and including the stack unit illustrated in Fig. 11 was used as a micromixer.

In the micromixer 1, a first plate 5 in which a first microtubular flow path is formed by a dry etching process, a second plate 7 in which a second microtubular flow path is formed by an etching process, and two temperature control plates 12 are alternately stacked as illustrated in Fig. 11. The resulting stacked body is further sandwiched between two cover plates. The plates are composed of SUS 304. The plate 5 and the plate 7 each have a thickness of 0.4 mm. The thickness of each of the temperature control plates 12 is 1.0 mm. The cross-sectional dimensions of each microtubular flow path of the first plate 5 are 0.4 mm in width, 0.2 mm in depth, and 40 mm in length, and the number of microtubular flow paths is 10. The cross-sectional dimensions of the large diameter portion 8a of a microtubular flow path of the second plate 7 are 1.2 mm in width, 0.2 mm in depth, and 20 mm in length. The cross-sectional dimensions of the small diameter portion 8b of each microtubular flow path of the second plate 7 are 0.4 mm in width, 0.2 mm in depth, and 2 mm in length, and the number of microtubular flow paths is 10. The cross-sectional dimensions of each of the temperature control plates 12 are 1.2 mm in width, 0.5 mm in depth, and 36 mm in length. The mixing space has an area of 100 mm² and a volume of 300 mm³.

In Example 1, a urethane acrylate was synthesized using the reaction device illustrated in Fig. 13 to evaluate a mixing efficiency of a first fluid and a second fluid. In Fig. 13, an outlet of a tank 61 for containing the first fluid is connected to an inlet of a plunger pump 63 through a pipe through which the first fluid is passed. An outlet of a tank 62 for containing the second fluid is connected to an inlet of a plunger pump 64 through a pipe through which the second fluid is passed. A pipe through which the first fluid is passed through the plunger pump 63 extends from an outlet of the plunger pump 63. A pipe through which the second fluid is passed through the plunger pump 64 extends from an outlet of the plunger pump 64. These pipes are connected to an inlet of the micromixer 1.

The first fluid and the second fluid are mixed in the micromixer 1 to form a fluid mixture. The fluid mixture is connected to a microreactor 65 or 66 for a chemical reaction through a pipe connected to the micromixer 1 or 2, the microreactors 65 and 66 being connected to the micromixer in parallel through a connector 67. The fluid mixture mixed in the micromixer 1 is distributed to the microreactor 65 or 66 for a chemical reaction in accordance with the time at which the fluid mixture is produced. The distributed mixed solution is reacted in the microreactor, and the resulting reaction product is discharged to a receiving container 68 or 69 through a pipe connected to an outlet.

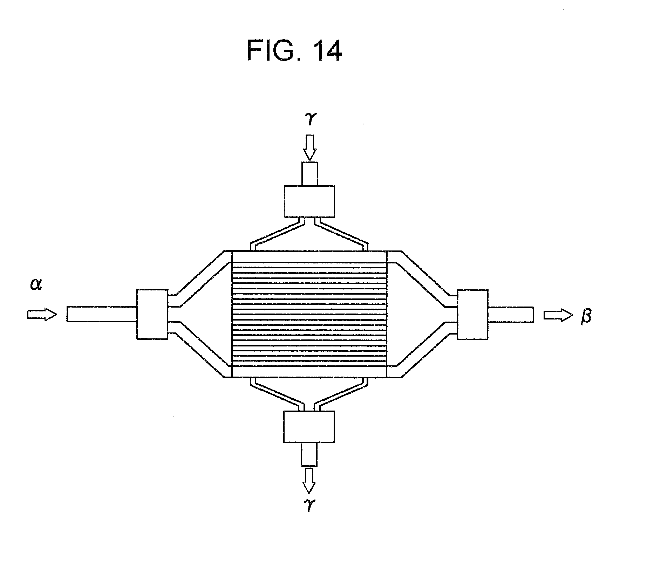

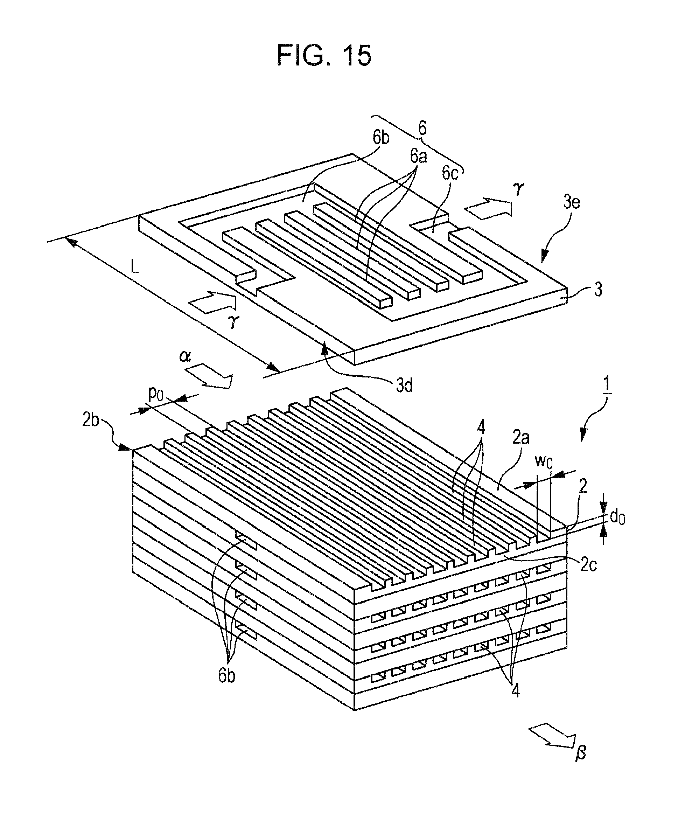

The microreactor 65 or 66 for a chemical reaction is a microreactor illustrated in Fig. 14. A stack unit in which a reaction is performed has a structure illustrated in Fig. 15. In Fig. 14, α, indicates a fluid mixture of the first fluid and the second fluid, β indicates a reaction product of the fluid mixture, and γ indicates a heat exchange medium.

In the microreactor 65 or 66 for a chemical reaction, two process plates in which five reaction flow paths 4 are formed by a dry etching process and three temperature control plates in which five temperature control flow paths 6 are formed by an etching process are alternately stacked. The process plates 2 and the temperature control plates 3 are composed of SUS 304 and each have a thickness of 1 mm. The cross-sectional dimensions of each of the reaction flow paths 4 and temperature control flow paths 6 are 1.2 mm in width and 0.5 mm in depth. The reaction flow paths 4 each have a length of 40 mm.

### <Device used in Example 2>

A device used in this Example was the same as the device used in Example 1. except that the micromixer 2 was used instead of the micromixer 1. A urethane acrylate was synthesized using this device to evaluate a mixing efficiency of a first fluid and a second fluid.

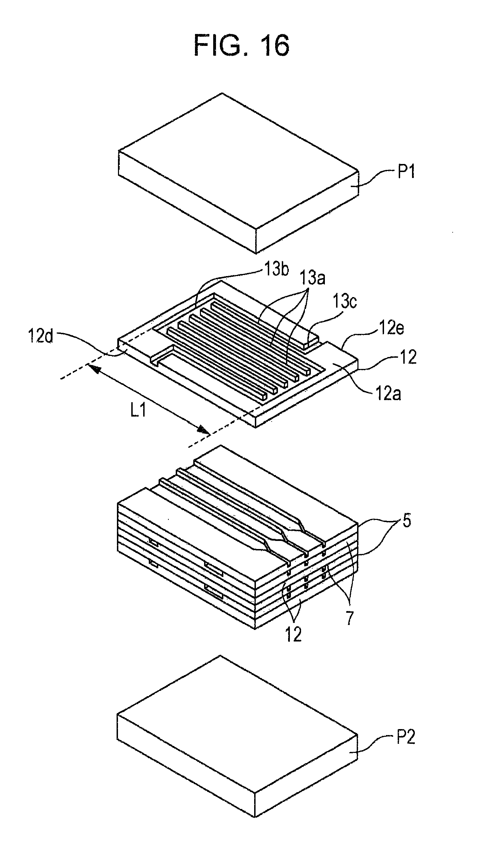

The micromixer 2 includes a stack unit illustrated in Fig. 16. In the micromixer 2, a first plate 5 in which a first microtubular flow path is formed by a dry etching process, a second plate 7 in which a second microtubular flow path is formed by an etching process, and two temperature control plates 12 are alternately stacked as illustrated in Fig. 14. The resulting stacked body is further sandwiched between two cover plates. The plates are composed of SUS 304. The plate 5 and the plate 7 each have a thickness of 0.4 mm. The thickness of each of the temperature control plates 12 is 1.0 mm. The mixing space has an area of 100 mm² and a volume of 300 mm³.

The first microtubular flow path 6 includes a large diameter portion 6f having a width of 1.2 mm, a depth of 0.2 mm, and a length of 38 mm and a small diameter portion 6g having a width of 0.4 mm, a depth of 0.2 mm, and a length of 2 mm. The number of microtubular flow paths in the first plate 5 is 10.

In the second plate 7, a microtubular flow path includes a large diameter portion 8a having cross-sectional dimensions of 1.2 mm in width, 0.2 mm in depth, and 20 mm in length. The microtubular flow path includes a small diameter portion 8b having a width of 0.4 mm, a depth of 0.2 mm, and a length of 2 mm, and the number of microtubular flow paths is 10. The cross-sectional dimensions of each of the flow paths of the temperature control plates 12 are 1.2 mm in width, 0.5 mm in depth, and 36 mm in length (L1).

### <Device used in Example 3>

A device used in Example 3 was a device in which a tank 61 for containing a first fluid, a tank 62 for containing a second fluid, plunger pumps, and a micromixer 3 were connected. Specifically, an outlet of the tank 61 for containing the first fluid is connected to an inlet of a plunger pump 63 through a pipe through which the first fluid is passed. An outlet of the tank 62 for containing the second fluid is connected to an inlet of a plunger pump 64 through a pipe through which the second fluid is passed. A pipe through which the first fluid is passed through the plunger pump 63 extends from an outlet of the plunger pump 63. A pipe through which the second fluid is passed through the plunger pump 64 extends from an outlet of the plunger pump 64. These pipes are connected to an inlet of the micromixer 3.

The first fluid and the second fluid are mixed in the micromixer 3 to form a fluid mixture. The fluid mixture is discharged to a receiving container through a pipe connected to the micromixer 3.

The micromixer 3 has a structure in which the temperature control plates 12 are omitted in Fig. 11. Specifically, a first plate 5 in which a first microtubular flow path is formed by a dry etching process and a second plate 7 in which a second microtubular flow path is formed by an etching process are stacked. The resulting stacked body is further sandwiched between two cover plates. The plates are composed of SUS 304. The plate 5 and the plate 7 each have a thickness of 0.4 mm. The first plate 5 includes a microtubular flow path having cross-sectional dimensions of 0.4 mm in width, 0.2 mm in depth, and 40 mm in length, and the number of microtubular flow paths is 1. In the second plate 7, a microtubular flow path includes a large diameter portion 8a having cross-sectional dimensions of 1.2 mm in width, 0.2 mm in depth, and 20 mm in length. The microtubular flow path includes a small diameter portion 8b having a width of 0.4 mm, a depth of 0.2 mm, and a length of 2 mm, and the number of microtubular flow paths is 1.

### <Device used in Example 4>

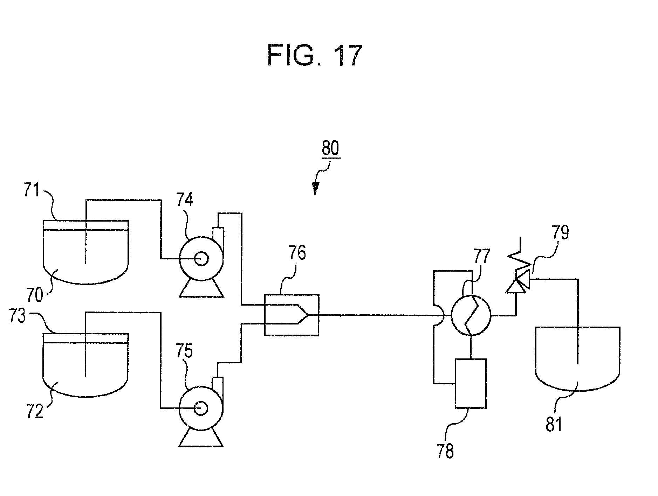

A reaction device illustrated in Fig. 17 was used in Example 4. In this device, the micromixer 1 used in Example 1 was used as a micromixer

A tank for containing a sulfuric acid solution, a tank for containing water, a micromixer, a heat exchanger, and a tank for collecting a dispersion containing the resulting pigment fine particles were connected as illustrated in Fig. 17 and used as a production device. A heat exchanger denoted by reference numeral 69 is a microreactor illustrated in Fig. 14. A stack unit in which a reaction is performed has a structure illustrated in Fig. 15. In the heat exchanger 69, five process plates in which five reaction flow paths 4 are formed by a dry etching process and seven temperature control plates in which five temperature control flow paths 6 are formed by an etching process are alternately stacked. The process plates 2 and the temperature control plates 3 are composed of SUS 304 and each have a thickness of 1 mm. The cross-sectional dimensions of each of the reaction flow paths 4 and the temperature control flow paths 6 are 1.2 mm in width and 0.5 mm in depth. The reaction flow paths 4 each have a length of 40 mm.

### EXAMPLE 1

A urethane acrylate was synthesized using an isocyanate compound whose viscosity was adjusted to 2 mPa·s as a first fluid and a multifunctional acrylate whose viscosity was adjusted to 300 mPa·s as a second fluid. As for an adjustment, a heat medium was supplied to the temperature control plates 12 to adjust the temperature of each of the fluids to 60°C.

The above compounds were allowed to flow to the micromixer 1 at a flow rate of 100 g/min with the plunger pumps 63 and 64 to obtain a fluid mixture. The resulting fluid mixture was divided into an initial fluid mixture and a later fluid mixture, and the two fluid mixtures were respectively supplied to the microreactors 65 and 66 by operating the connector. In each of the microreactors, a reaction was conducted at 160°C for a retention time of 50 seconds. Urethane acrylates obtained by using the microreactor 65 and 66 are respectively abbreviated as a urethane acrylate (1-1) and a urethane acrylate (1-2). According to the results of gel permeation chromatography (GPC), in each of the urethane acrylate (1-1) and the urethane acrylate (1-2), the area ratio [%] of a urethane acrylate and the area ratio [%] of a residual acrylate were 95 [%] and 1.3 [%], respectively, and thus the same results were obtained. These results show that the fluids were satisfactorily mixed in the micromixer 1.

### EXAMPLE 2

A urethane acrylate was synthesized using an isocyanate compound whose viscosity was adjusted to 100 mPa·s as a first fluid and a multifunctional acrylate whose viscosity was adjusted to 300 mPa·s as a second fluid. As for an adjustment, a heat medium was supplied to the temperature control plates 12 to adjust the temperature of each of the fluids to 60°C.

The above compounds were allowed to flow to the micromixer 1 at a flow rate of 100 g/min with the plunger pumps 63 and 64 to obtain a fluid mixture. The resulting fluid mixture was divided into an initial fluid mixture and a later fluid mixture, and the two fluid mixtures were respectively supplied to the microreactors 65 and 66 by operating the connector. In each of the microreactors, a reaction was conducted at 160°C for a retention time of 50 seconds. Urethane acrylates obtained by using the microreactors 65 and 66 are respectively abbreviated as a urethane acrylate (1-1) and a urethane acrylate (1-2). According to the results of gel permeation chromatography (GPC), in each of the urethane acrylate (1-1) and the urethane acrylate (1-2), the area ratio [%] of a urethane acrylate and the area ratio [%] of a residual acrylate were 95.5 [%] and 1.3 [%], respectively, and thus the same results were obtained. These results show that the fluids were satisfactorily mixed in the micromixer 2.

In Example 1, the flow rate of the first fluid in the outlet portion of the microtubular flow path in the mixing space of the micromixer of the present invention was 0.42 m/s, and the flow rate of the second fluid in the outlet portion of the microtubular flow path was 1.66 m/s.

### EXAMPLE 3

Water having a viscosity of 1 mPa·s and a starch syrup having a viscosity of 30 mPa·s were allowed to flow to a device having a micromixer at a flow rate of 10 g/min with plunger pumps. As a result, it was confirmed that the water and the starch syrup could be mixed without pulsation.

### EXAMPLE 4

A sulfuric acid solution (viscosity at room temperature: 27 mPa·s) was prepared by mixing 7 g of 98 mass% concentrated sulfuric acid with 1,771 g of C.I. Pigment Red 177 serving as a pigment having an anthraquinone structure (the content of the pigment in the sulfuric acid solution: 12.5% by mass). In dissolving the pigment, the liquid temperature was 37°C and the stirring time was two hours. One hundred grams of the sulfuric acid solution was put in the tank 62 of the device illustrated in Fig. 17 and 1,500 g of water was put in the tank 64.

The sulfuric acid solution in the tank 62 and the water in the tank 64 were fed to the micromixer 67 using the plunger pumps 65 and 66 so that the weight ratio was 1:10 and the flow rate was 88 g/min. A fluid mixture containing pigment fine particles was prepared in the micromixer At this time, the liquid temperature (T1) of the fluid mixture was 40°C. Next, the mixed solution was transferred to the heat exchanger, and a dispersion of the pigment fine particles at a liquid temperature (T2) of 25°C was obtained. The time required for adjusting the liquid temperature to the temperature (T2) was 15 seconds from the starting point when the mixed solution (A) was obtained. The water was allowed to flow through the microtubular flow path of the first blade, and the sulfuric acid solution was allowed to flow through the microtubular flow path of the first blade.

The pigment fine particles in the dispersion 1 of the pigment fine particles had an average particle diameter (SAXS particle diameter measured by a small-angle scattering method) of 10.1 nm.

### COMPARATIVE EXAMPLE 1

An experiment of mixing fluids having different viscosities was conducted as in Example 3 except that a Y-shaped micromixer was used instead of the micromixer 3. Regarding the Y-shaped micromixer, a flow path through which a first fluid flows had a width of 0.4 mm, a depth of 0.2 mm, and a length of 15 mm. A flow path through which a second fluid flows also had a width of 0.4 mm, a depth of 0.2 mm, and a length of 15 mm. A flow path through which the resulting liquid mixture flows had a width of 0.8 mm, a depth of 0.2, and a length of 15 mm. The plate having the flow paths is composed of the same material as that of the micromixer 1. Accordingly, the roughness of each of the wall surfaces of the flow paths is the same as that of the micromixer 1.

According to the experimental results, it was confirmed that an intermittent flow was observed in water, which has a low viscosity, and thus it was difficult to realize a stable flow. The reason for this is believed to be as follows. The Y-shaped micromixer is designed so that the flow paths intersect and the fluids join together. Therefore, when two types of fluids having different viscosities are allowed to flow through the flow paths, the fluids flow such that the pressure loss of a low-viscosity fluid and the pressure loss of a high-viscosity fluid become the same. In this experiment, the area of the cross section of a confluence portion is 0.8 mm × 0.2 mm in depth = 0.16 mm². The area of the cross section through which the high-viscosity fluid flows is 0.16 × 30/31 = 0.155 mm² whereas the area of the cross section through which the low-viscosity fluid flows is 0.16 × 1/31 = 0.005 mm². Thus, since the area of the cross section through which the low-viscosity fluid flows is very small, the intermittent feeding occurs.

### COMPARATIVE EXAMPLE 2

A urethane acrylate (2'-1) and a urethane acrylate (2'-2) were prepared as in Example 1 except that the micromixer used in Example of Japanese Unexamined Patent Application Publication (Translation of PCT Application) No. 9-512742 was used instead of the micromixer 1, and the fluids were allowed to flow with plunger pumps so that the flow rate was 12.5 g/min. Microtubular flow paths of a first plate and a second plate of the micromixer used in Example of Japanese Unexamined Patent Application Publication (Translation of PCT Application) No. 9-512742 each have cross-sectional dimensions of 0.4 mm in width, 0.2 mm in depth, and 40 mm in length. The number of the microtubular flow paths in each of the plates is 10, and one first plate and one second plate are stacked.

In the prepared urethane acrylate (2'-1) and urethane acrylate (2'-2), the area ratios [%] of a urethane acrylate were 92 [%] and 77 [%], respectively, and the area ratios [%] of a residual acrylate were 2.7 [%] and 18.8 [%], respectively. Thus, the area ratios were different from each other. According to these results, it was concluded that urethane acrylates having different physical property values were produced, and uniform mixing was not conducted. Furthermore, although the fluids were allowed to flow such that the flow rate was 100 g/min, the pressure loss of the multifunctional acrylate whose viscosity was adjusted to 300 mPa·s became 10 MPa or more because of the high viscosity, it was difficult to allow the fluid to flow, and thus a urethane acrylates could not be obtained.

### Reference Signs List

C: case of micromixer 1
C1: left end of case C
C2: lower right end of case C
C3: lower left end of case C
C4: upper end of case C
F1: first fluid
F2: second fluid
F3: fluid mixture of first fluid and second fluid
H1: heat medium
S1: storage portion that temporarily stores first fluid (F1)
S2: storage portion that temporarily stores second fluid (F2)
S3: mixing space
1A: first fluid supply unit
1B: opening formed at left end of case C
1C: connector connected to opening 2B
2A: second fluid supply unit
2B: opening formed at lower right end of case C
2C: connector connected to opening 2B
3A: heat medium supply unit that supplies heat medium H1 to case C
3B: opening formed at lower left end of case C
3C: connector connected to opening 3B
4A: heat medium-sending unit
4B: opening formed at upper right end of case C
4C: connector connected to opening 4B
5: first plate
5A: sending unit including opening 5B and connector 5C
5B: opening formed at right end of case C
5C: connector connected to opening 5B
6: first microtubular flow path
6A: first-microtubular-flow-path-forming portion
6a: top surface of first-microtubular-flow-path-forming portion 6A
6b: left-side end of first-microtubular-flow-path-forming portion 6A
6c: right-side end of first-microtubular-flow-path-forming portion 6A
6d: inlet of first microtubular flow path 6
6e: outlet of first microtubular flow path 6
7: second plate
7A: second-microtubular-flow-path-forming portion
7a: top surface of second-microtubular-flow-path-forming portion 7A
7b: lower-side end of second-microtubular-flow-path-forming portion 7A
7c: end extending from lower-side end 7b of second-microtubular-flow-path-forming portion 7A in short direction
7d: right-side end of second-microtubular-flow-path-forming portion 7A
8: second microtubular flow path
8a: inlet of second microtubular flow path 8
8b: outlet of first microtubular flow path 6
9: large diameter portion having large flow-path diameter in second microtubular flow path 8
10: small diameter portion having small flow-path diameter in second microtubular flow path 8
11: tapered portion in second microtubular flow path 8
12: temperature control plate
12a: surface of temperature control plate 12
13: temperature control flow path that has cross section having recessed groove shape and that is provided in surface 12a of temperature control plate 12
13a: main flow paths arranged in the longitudinal direction of temperature control plate 12
13b: supply-side flow path connected to main flow paths 13a
13c: discharge-side flow path connected to main flow paths 13a
61: tank for containing first fluid
62: tank for containing second fluid
63: plunger pump
64: plunger pump
65: tube reactor
66: tube reactor
67: connector
68: receiving container
69: receiving container
70: sulfuric acid solution of pigment
71: first tank
72: water
73: second tank
74: plunger pump
75: plunger pump
76: micromixer
77: temperature control unit
78: heat exchanger for cooling
79: exhaust pressure valve
80: reaction device
81: receiving container
110: stacked body

## Claims

1. A micromixer comprising a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows; and a mixing space which is connected to an outlet of the first microtubular flow path and an outlet of the second microtubular flow path and in which the first fluid and the second fluid are mixed, wherein, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion.

2. The micromixer according to Claim 1,
wherein, in the first plate, a cross-sectional area of the microtubular flow path in the outlet portion connected to the mixing space is smaller than a cross-sectional area of the microtubular flow path in the inlet portion of the microtubular flow path connected to the fluid supply path, and,
in the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion.

3. The micromixer according to Claim 1, wherein the single microtubular flow path in the inlet portion has a cross-sectional area of 0.01 to 40 mm², and the microtubular flow path in the outlet portion has a cross-sectional area of 0.01 to 10 mm².

4. The micromixer according to Claim 1, wherein the stack unit further includes a temperature control plate having a heat exchange medium flow path through which a heat exchange medium flows.

5. The micromixer according to Claim 1, wherein the area of a cross section at which the mixing space is connected to the outlet of the first microtubular flow path and the outlet of the second microtubular flow path is 10 to 200 times the sum of the cross-sectional area of the cutlet of the first microtubular flow path and the cross-sectional area of the outlet of the second microtubular flow path, and a distance between the outlet portion of the first microtubular flow path and an outlet portion of the mixing space through which a mixture of the first and second fluids flows and between the outlet portion of the second microtubular flow path and the outlet portion of the mixing space is 2 to 100 mm.

6. A method for producing a liquid mixture using a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion, the method comprising allowing the first fluid and the second fluid that have a viscosity ratio of 10 to 3,000 to respectively flow through the first microtubular flow path and the second microtubular flow path to obtain a fluid mixture containing the first fluid and the second fluid in the mixing space.

7. A method for producing a urethane (meth) acrylate using a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion,
the method comprising:
a first step of allowing a compound (A) having an isocyanate group to flow through one of the first microtubular flow path and the second microtubular flow path and allowing a (meth)acrylate (B) having a hydroxyl group to flow through the other microtubular flow path to obtain a fluid mixture containing the compound (A) and the compound (B) in the mixing space; and
a second step of allowing the compound (A) and the compound (B) in the fluid mixture to react with each other.

8. A method for producing pigment fine particles having an anthraquinone structure using a micromixer including a stack unit in which a second plate is stacked on a first plate, the second plate having a second microtubular flow path through which a second fluid flows and which is connected to a fluid supply path through which the second fluid flows, the first plate having a first microtubular flow path connected to a fluid supply path through which a first fluid flows, and a mixing space which is connected to an outlet of the first microtubular flow path and an outlet of the second microtubular flow path and in which the first fluid and the second fluid are mixed, in which, in at least one of the first plate and the second plate, the microtubular flow path has an inlet portion connected to the fluid supply path and constituted by a single flow path, and a cross-sectional area of the microtubular flow path in an outlet portion connected to the mixing space is smaller than a cross-sectional area of the microtubular flow path in the inlet portion,
the method comprising:
a first step of mixing water (C) and a sulfuric acid solution (D) of a pigment having an anthraquinone structure in the mixing space by allowing the water (C) to flow through one of the first microtubular flow path and the second microtubular flow path and allowing the sulfuric acid solution (D) to flow through the other microtubular flow path to obtain a fluid mixture in which the pigment having an anthraquinone structure is deposited; and
a second step of cooling the fluid mixture in which the pigment is deposited.
